(19) **Europäisches Patentamt**
**European Patent Office**
**Office européen des brevets**

(11) **EP 1 864 674 B1**

(12) **EUROPEAN PATENT SPECIFICATION**

(45) Date of publication and mention
of the grant of the patent:
**07.08.2013 Bulletin 2013/32**

(21) Application number: **06733214.8**

(22) Date of filing: **17.02.2006**

(51) Int Cl.:
*A61K 33/24* (2006.01)      *A61K 35/10* (2006.01)
*A61K 36/00* (2006.01)      *A61P 39/00* (2006.01)

(86) International application number:
**PCT/RU2006/000078**

(87) International publication number:
**WO 2006/088394 (24.08.2006 Gazette 2006/34)**

(54) **METHOD FOR PRODUCING MEANS PROTECTING AN ORGANISM AGAINST IONISING RADIATION**

VERFAHREN ZUR HERSTELLUNG EINES MITTELS ALS SCHUTZ EINES ORGANISMUS VOR IONISIERENDER STRAHLUNG

PROCEDE DE FABRICATION DE PRODUITS DE PROTECTION CONTRE LES RAYONNEMENTS IONISANTS

(84) Designated Contracting States:
**AT BE BG CH CY CZ DE DK EE ES FI FR GB GR HU IE IS IT LI LT LU LV MC NL PL PT RO SE SI SK TR**

(30) Priority: **17.02.2005 RU 2005104289**

(43) Date of publication of application:
**12.12.2007 Bulletin 2007/50**

(73) Proprietor: **Rdinnovation APS**
**1360 (DK)**

(72) Inventors:
• **SHIPOV, Valery Pavlovich**
**St-Petersburg, 194156 (RU)**
• **PIGAREV, Evgeny Sergeevich**
**St. Petersburg, 193967 (RU)**
• **FEDOROS, Elena Ivanovna**
**St. Petersburg, 193315 (RU)**
• **TROFIMOVA, Nadezhda Petrovna**
**St. Petersburg, 194156 (RU)**

(74) Representative: **Scholz, Volker et al**
**Boehmert & Boehmert**
**Pettenkoferstrasse 20-22**
**80336 München (DE)**

(56) References cited:
**RU-C1- 2 022 968      RU-C1- 2 034 558**
**RU-C1- 2 164 411      RU-C1- 2 183 124**

**Description**

Field of invention

The invention belongs to methods for producing of natural substance-based means for organism protection from ionizing radiations, or, more precisely, to methods for producing of such substances from humic compounds, and may be used in the development of pharmaceuticals for mitigation of harmful consequences of external radiation exposure of living beings and acceleration of excretion of heavy metals (including radioactive ones) from the organism.

Prior art

Substances able to protect a live organism from external effects of ionizing radiation are usually called radioprotectors. As a rule, radioprotectors are efficient when administered within a short period (usually 10-30 minutes) prior to the radiation exposure.

Among radioprotectors, currently considered most efficient are two classes of compounds: mercaptoalkylamines and indolylalkylamines [1]. (1. E.N. Goncharenko, Yu.B. Kudryashov. Chemical protection against radiation injury. Moscow, Moscow State University, 1985, 147 pp. (in Russian).

Despite their proven efficiency, the use of these preparations is limited by their high toxicity.

Furthermore, these preparations, like many other radioprotectors, exhibit low efficiency when used after the radiation exposure (for medicinal / therapeutic purpose).

Special methods for protection against radiation injury include the measures to accelerate excretion of radionuclides having entered the body and present in its organs and tissues. Radionuclides incorporated into the body cause a so-called internal irradiation, which has certain distinctions from external one, when the source of radiation affects the organism from the outside environment. In particular, the danger of internal irradiation is caused not only by gamma radiation with its high penetrating power (as in the case of external exposure), but also by beta and especially alpha radiation, less penetrating into the body in case of external irradiation. Radiation injuries in case of internal irradiation also depend on the half-life of the radionuclide, its distribution in the tissues and the rate of excretion from the body.

Besides mechanical methods (frequent sauna to increase perspiration, gastrointestinal colonic lavage, etc.), preparations (usually absorbents and complexing agents) are used to bind the radionuclides into structures rapidly excreted from the body.

Previously known is the use of humic substances for decontamination of radionuclide-contaminated soil, e.g. the method described in (A, Russian Federation, No. 208898). The method involves treatment of the object in question with water containing acids of humic origin (humic substances) with subsequent removal of radioactive contaminants from the solution. The treatment is effected using natural waters of humid climatic areas containing at least 60 mg/l fulvic acids, with pH from 3.5 to 6.5.

The authors of the present document discovered no indications of the use of humic substances for elimination of radionuclides from an organism.

Previously known is a method for producing means protecting an organism against ionizing radiations, namely a substance with radioprotective properties (Russian Federation patent No. 2183124) from materials of natural origin, according to which humic substances are derived from natural raw materials, an aqueous solution of such humic substances being subsequently treated with ammonium molybdate. The said ammonium molybdate treatment is conducted at the temperature of 40±5°C under irradiation with ultrasonic waves with the power density of 40 W/cm$^3$ and the frequency of 22 kHz for 4 to 8 minutes. The method uses humic substances obtained from oxidized wood ligning.

The substance obtained was tested for radioprotective efficiency. The research has demonstrated that the substance obtained is effective as a radioprotector in case of relatively uniform generalized irradiation of mice in the bone marrow-affecting and intestine-affecting dose ranges.

It has been shown experimentally that the most active among humic substances are water-soluble substances with the molecular weight in the 500-10000 dalton range. Components of higher molecular weight are less active because of low solubility and very poor uptake by the body.

In concentrated (over 0.5-1%) solutions, humic substances undergo frequent intermolecular interactions, namely the reactions of condensation and polymerization, which result in an increase in the average molecular weight.

This leads to inhomogeneity of the product, precipitate fallout, changes in physico-chemical properties, and instability of the preparations in storage. Thus, when stored under artificial aging conditions for as short a period as 10-20 days, the preparation obtained by the known method exhibited the presence of foreign inclusions (high molecular weight compounds) and color changes attesting to degradation of physico-chemical properties of the preparation.

The present invention is based upon the problem of developing a method for production of a preparation for protection against ionizing radiations allowing to produce a more homogeneous preparation with a better stability in storage by reducing the relative content of high molecular weight compounds.

The problem is solved using the method for production of a drug for protection from ionizing radiations based on humic substances as defined in claim 1 in which the aqueous solution containing humic substances and ammonium molybdate is treated with wave radiation; according to the invention, the content of ammonium molybdate is selected in the range up to 0.4 parts by weight per 1 part of humic substances, the treatment being conducted until the high molecular fraction of humic substances is decreased to the level of 5% or less.

It has been established experimentally that the reduction of high molecular fraction below the said level allows for a substantial improvement of preparation homogeneity and its stability in storage.

Waves for irradiation may be reasonably selected in the ultrasonic range, with frequencies from 18 kHz to 66 kHz and power density from 0.5 to 5 W/cm$^3$.

The treatment procedure is the most efficient at the irradiation power density of 5 W/cm$^3$ at the frequency of 22 kHz for 5 to 20 minutes.

Irradiation may also be performed with microwaves in the frequency range from 0.3 to 30 GHz and power from 0.5 to 50 kW; for the most efficient treatment at the irradiation power density of 5 W/cm3 at the frequency of 2.45 GHz, the product being treated is maintained at the temperature of 60-70°C for 30 to 90 minutes.

Humic substances are most expediently produced from oxidized wood lignin, which allows to achieve good reproducibility of results.

The invention is illustrated by the drawings, on which:

Fig. 1 presents the dynamics of copper content in the blood of mice during the use of preparations produced by a previously known method and the method according to the invention;
Fig. 2 presents the dynamics of copper content in the urine of mice during the use of preparations produced by a previously known method and the method according to the invention;
Fig. 3 presents the properties of humic substances as a function of treatment duration for a given treatment regime;
Fig. 4 presents the properties of humic substances as a function of treatment duration for another given treatment regime;

Implementation examples for the method according to the invention are described below.

Example 1.

Several preparations were produced in order to compare the properties of preparations produced by the known method vs. the claimed method.

All the preparations were produced by wave irradiation of aqueous solution of humic substances.

For Preparation 1 (Prep. 1 in the tables), during the wave irradiation according to the prototype method, the aqueous solution of humic substances was doped with 0.4 g ammonium molybdate powder per 1 g humic substances.

Preparation 2 (Prep. 2 in the tables) was obtained in the same manner, but at the 0.2:1 ration of ammonium molybdate to humic substances.

Preparation 3 (Prep. 3 in the tables) contained no ammonium molybdate. Radioprotective efficiency of the substances produced by the suggested method was tested on male mongrel mice weighing 18 to 20 g.

Irradiation was conducted by an X-ray machine with the following parameters: 180 kV voltage, 15 mA, current, 0.5 mm Cu + 1.0 mm Al filter, 70 cm focal length, 0.355 Gy/min dose rate in the dorsoventral direction. Different groups of animals received absorbed radiation doses of 2, 4, 6, 8, 10, 15 and 20 Gy.

To perform irradiation, groups of 10 mice were placed in plastic containers. The efficiency of irradiation was controlled using false irradiation, where the animals in plastic containers were placed under the X-ray machine with anode voltage off for the same period as actual irradiation. Test and control groups of animals were irradiated simultaneously and subsequently kept under standard conditions. Dose control was conducted using personal dosimeters.

The substances produced (Preparations 1, 2 and 3) in the form of 1% solution in the normal saline solution were administered to test animals by 0.2 ml intramuscularly (resulting in the dose of 100 mg/kg, or 2 mg per animal). The preparations were injected on a daily basis 5, 4, 3 and 2 days before the irradiation. The total dose of the preparations for 4 days was 400 mg/kg (8 mg per animal).

In order to study radiomodifying efficiency of the preparations in question, control groups of mice were injected with normal saline solution by the same scheme, whereupon the mice were subjected to irradiation as described above.

The animals in the test and control groups were kept under observation for one month before irradiation and 30 days after irradiation.

The research has demonstrated that the substance obtained is effective as a radioprotector in case of relatively uniform generalized irradiation of mice in the bone marrow-affecting and intestine-affecting dose ranges.

The results of the experiment are presented in Tables 1-4.

Table 1 presents the dynamics of mouse mortality after irradiation in different doses. Table 2 presents mortality and survival of mice after irradiation in different doses along with radioprotective efficiency data for the preparations in question.

Table 1

| Dose, Gy | Preparation | Mice count | Study time after irradiation, days | | | | | | | | | | | | | | | | |
|---|---|---|---|---|---|---|---|---|---|---|---|---|---|---|---|---|---|---|---|
| | | | 3 | 4 | 5 | 6 | 8 | 9 | 10 | 11 | 12 | 13 | 14 | 15 | 18 | 20 | 21 | 26 | 30 |
| 4 | Control | 10 | | | | | | | | | | 1 | | | 1 | | | | |
| | Prep. 1 | 10 | | | | | | | | | | | | | | | 1 | | |
| | Prep. 2 | 10 | | | | | | | | | | | | | | | 1 | | |
| | Prep. 3 | 10 | | | | | | | | | | | | | 1 | | | | |
| 6 | Control | 10 | | | | | | | 1 | 1 | 2 | 1 | 1 | | | | | | |
| | Prep. 1 | 10 | | | | | | | | | | | 1 | | | | 1 | | |
| | Prep. 2 | 10 | | | | | | | | | 1 | | | | | 1 | | | |
| | Prep. 3 | 10 | | | | | | | | | | | 1 | 1 | | | | | |
| 8 | Control | 10 | 1 | 2 | | | | | | 3 | 2 | 1 | | | | | | | |
| | Prep. 1 | 10 | | | | 1 | | | | | 1 | 1 | 2 | 1 | | | 2 | | |
| | Prep. 2 | 10 | | | 1 | | | | | 1 | 1 | 2 | 2 | | | 1 | 1 | | |
| | Prep. 3 | 10 | | 1 | | 1 | | | | 3 | 1 | 2 | | | 1 | | | | |
| 10 | Control | 10 | 1 | 9 | | | | | | | | | | | | | | | |
| | Prep. 1 | 10 | | 1 | 4 | 1 | 1 | 3 | | | | | | | | | | | |
| | Prep. 2 | 10 | 2 | 2 | 2 | 1 | 4 | | | | | | | | | | | | |
| | Prep. 3 | 10 | 2 | 3 | 1 | 4 | | | | | | | | | | | | | |

Table 2

| Dose, Gy | Preparation | % mortality | % survival | Average survival time for the animals lost | % protection | Survival index | Protection coefficient |
|---|---|---|---|---|---|---|---|
| 4 | Control | 20 | 80 | 16 | | | |
| | Prep. 1 | 10 | 90 | 21 | 10 | 1,125 | 0,5 |
| | Prep. 2 | 10 | 90 | 21 | 10 | 1,125 | 0,5 |
| | Prep. 3 | 10 | 90 | 20 | 10 | 1,125 | 0,5 |
| 6 | Control | 70 | 30 | 14 | | | |
| | Prep. 1 | 20 | 80 | 17 | 50 | 2,67 | 0,71 |
| | Prep. 2 | 20 | 80 | 16 | 50 | 2,67 | 0,71 |
| | Prep. 3 | 20 | 80 | 14,5 | 50 | 2,67 | 0,71 |
| 8 | Control | 100 | 0 | 9 | | | |
| | Prep. 1. | 80 | 20 | 14,5 | 20 | - | 0,2 |
| | Prep. 2 | 90 | 10 | 12,04 | 10 | - | 0,1 |
| | Prep. 3 | 90 | 10 | 11 | 10 | - | 0,1 |
| 10 | Control | 100 | 0 | 3,22 | | | |
| | Prep. 1 | 100 | 0 | 6,5 | 0 | - | 0 |
| | Prep. 2 | 100 | 0 | 5,14 | 0 | - | 0 |
| | Prep. 3 | 100 | 0 | 4,7 | 0 | - | 0 |

The following parameters were used as the criteria for assessing the radiomodifying efficiency of the preparations.

Mortality percentage was calculated by dividing the total number of mice lost by the total number in the group and multiplying the ratio by 100.

Survival percentage is the complementary value to the mortality percentage. Average survival time for the animals lost was calculated by adding up the number of days between irradiation and death of each animal lost, and dividing this figure by the total number of animals lost in the study group. Average survival time was expressed in days.

Protection percentage of the preparation was calculated as the difference in survival percentages between the study and control groups of animals.

Survival index (SI) was calculated as the ratio of survival percentage for the study group of mice to that for the control group.

Protection coefficient (Kp) was calculated as the ratio of the mortality percentage difference between the control and study groups to the mortality percentage in the control group:

$$Kp = (\text{\% mortality for control group} - \text{\% mortality for study group}) \, / \, \text{\% mortality for control group}$$

The dose modification factor (DMF) was calculated as the ratio of irradiating doses causing the same biological effect (in particular, LD50/30 and LD50/5) with the preparations proposed and without them:

$$DMF = LD50/30 \text{ with preparation} \, / \, LD50/30 \text{ without preparation.}$$

It can be seen from Table 1 that mortality of the animals receiving no pharmaceutical protection was detected starting from the dose of 4 Gy; for the dose of 8 Gy, no animals survived after irradiation. A determination of lethal dose indicators resulted in LD16/30 = 3.50 Gy, LD50/30 = 5.33 Gy, LD84/30 = 7.15 Gy.

Average survival time of the animals lost after irradiation with bone marrow-affecting doses varied from 16 days (for 4 Gy) to 9 days (for 8 Gy). After irradiation with intestine-affecting doses (10 Gy), 100% mortality was observed with the average survival time of 3.22 days.

As it can be seen from Table 2, preventive use of the preparations proposed led to a decrease in animal mortality. Upon preventive administration, the following mortality characteristics were obtained: LD16/30 = 4.71 Gy, LD50/50 = 6.56 Gy, LD84/30 = 8.75 Gy (Preparation 1); 8.41 Gy (Preparation 2); 8.26 Gy (Preparation 3).

The DMF of the preparations proposed amounted to 1.28 for the mice irradiated with the minimum lethal dose, 1.26 for the half-lethal dose, and 1.27, 1.25 and 1.24 (preparations 1, 2 and 3, respectively) for the minimum absolute lethal dose; thus, for the mice irradiated with bone marrow-affecting doses (4 to 8 Gy), DMF remained practically unchanged with a dose increase.

The preventive administration of the preparations produced has demonstrated the Preparation 1 (with ammonium molybdate in the ratio of 0.4:1 to humic substances) to have the highest radioprotective effect, increasing the survival time of the animals lost after 10 Gy irradiation by a factor of 2, whereas the Preparation 2 (per prototype method, with the 0.2:1 ratio of ammonium molybdate to humic substances) increases the survival time by a factor of 1.6, and Preparation 3 (without ammonium molybdate) - by a factor of 1.5.

**Example 2.**

The complexing properties of the substances produced (used for protection against intoxication with heavy metals, including radioactive ones) were tested on male mongrel mice weighing 18-20 g. Copper was selected as the heavy metal; it was administered to animals orally in the form of copper sulfate in a single dose of 500 mg/kg (approx. 10 mg per animal), which is equivalent to 200 mg/kg (or 4 mg per animal) of elementary copper. For administration, the calculated quantity of copper sulfate was dissolved in 0.5 ml of normal saline solution.

The control group received copper sulfate only. The study groups additionally received the substances produced in accordance with Example 1 (Preparation 1, Preparation 2 and Preparation 3). These substances in the form of 1% solution in the normal saline solution were administered to test animals by 0.2 ml intraperitoneally (resulting in the dose of 100 mg/kg, or 2 mg per animal). For rodents, intraperitoneal injection is equivalent to intravenous. The preparations were injected on a daily basis on the -1st, 0th, 1st and 2nd days of the experiment. The total dose of the preparations for 4 days was 400 mg/kg (8 mg per animal).

Trilon B (disodium ethylenediaminetetraacetate (EDTA)) was used as a reference preparation. The reference preparation was injected by the same scheme as the Preparations 1-3, 0.2 ml of 0.5% solution intraperitoneally on the -1st, 0th, 1st and 2nd days of the experiment.

The concentrations of copper in the blood and urine of animals was surveyed. The sampling scheme is presented in Table 3.

Table 3.

| Experiment scheme | | | | | | | | | | |
|---|---|---|---|---|---|---|---|---|---|---|
| Groups | Number of animals in the group | Biological material sampling time (hours after administration of copper sulfate) | | | | | | | | |
| | | 4 | 8 | 12 | 24 | 48 | 72 | 120 | 192 |
| Control | 32 | 4 | 4 | 4 | 4 | 4 | 4 | 4 | 4 |
| Preparation 1 | 32 | 4 | 4 | 4 | 4 | 4 | 4 | 4 | 4 |
| Preparation 2 | 32 | 4 | 4 | 4 | 4 | 4 | 4 | 4 | 4 |
| Preparation 3 | 32 | 4 | 4 | 4 | 4 | 4 | 4 | 4 | 4 |
| EDTA | 32 | 4 | 4 | 4 | 4 | 4 | 4 | 4 | 4 |

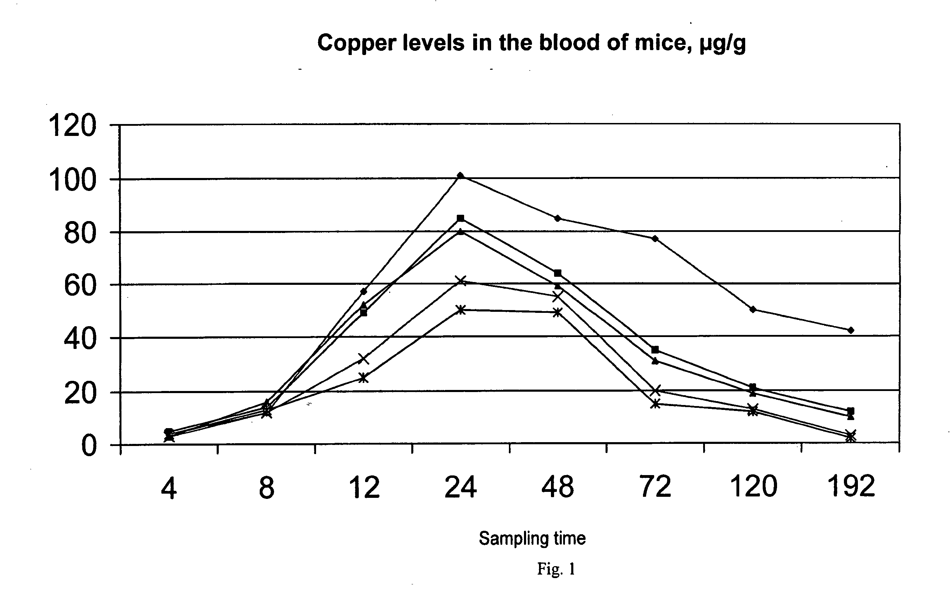

The dynamics of copper content in the blood of the study animals is presented in Fig. 1. The horizontal axis represents the sampling time, the vertical axis - copper level ($\mu$g/g) in the blood of mice. Curves 1, 2 and 3 present the dynamics of copper levels in the blood of mice administered with Preparations 1, 2 and 3, respectively, curve 4 presents the dynamics of copper levels in the blood of mice administered with EDTA, curve 5 presents the data for the control group. It has been demonstrated that blood concentrations of copper increase within the first 12-24 hours of the study, and then start to decrease. In the study groups, the blood copper levels were found to drop much faster than in the control group. Preparations 1 and 2 (with ammonium molybdate) were slightly less efficient than Preparation 3 (without ammonium molybdate).

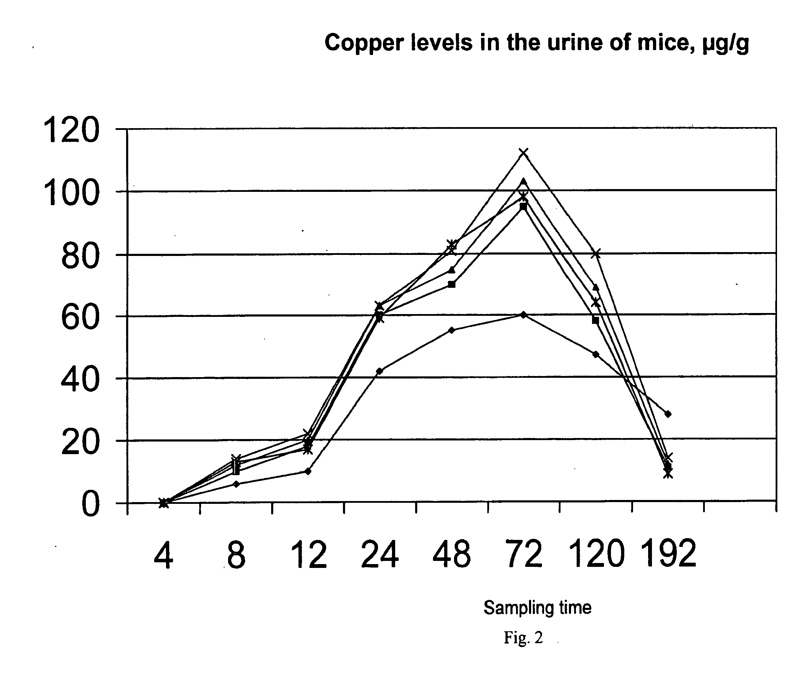

The dynamics of copper excretion with urine is shown in Fig. 2, with the horizontal axis representing the sampling time and the vertical axis representing the copper concentration in the urine of mice ($\mu$g/g). Curves 6, 7 and 8 show the dynamics for the Preparations 1, 2 and 3, respectively, curve 9 - for EDTA, curve 10 - for the control group. The curves reveal the heavy metal to be excreted faster and more completely in the study groups as compared to the control group. Preparations 1 and 2 (with ammonium molybdate) were slightly less efficient than Preparation 3 (without ammonium molybdate).

**Example 3.**

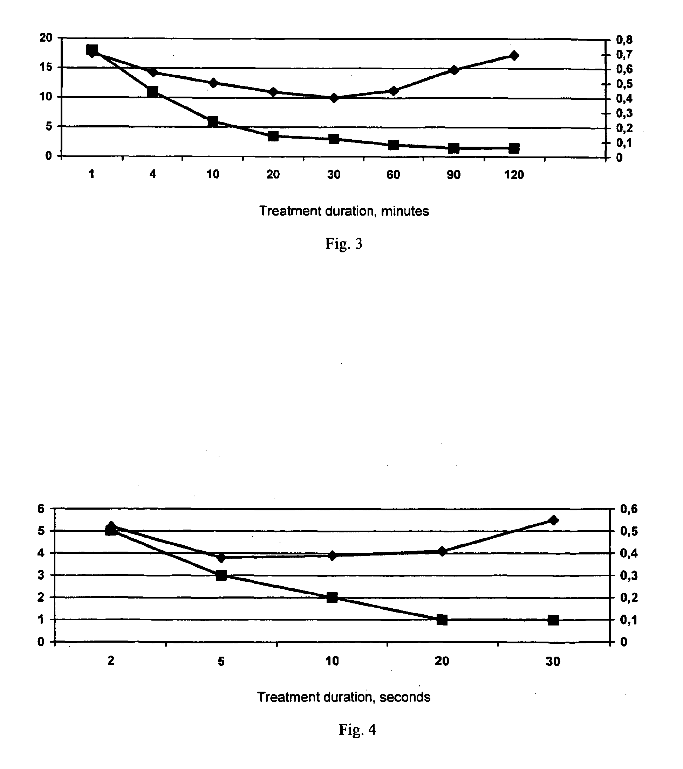

The preparations for research were produced in the same manner as in Examples 1 or 2, except for the duration of ultrasonic exposure. The exposure time varied from 1 to 180 minutes. The preparations for checked for their color index ($D_{445}$ / $D_{665}$), the changes in which indirectly attest to the progress of molecular condensation and polymerization processes, and for molecular weight. The color index was measured for 0.001% standardized concentration solutions using a spectrophotometer with a 10 mm cell. Molecular weight was assessed by means of thin-layer chromatography on Silufol plates by Chemapol (Czech Republic) using the standard eluent in the 1:4 concentration. The level of high molecular weight fraction (X, %) was assessed by comparing the color intensity of the starting spot with the residual spot after elution. The dependence of the properties of humic substances in the preparation on the duration of ultrasonic exposure at the power density of 0.5 W/cm$^3$ and the frequency of 22 kHz is presented in Fig. 3. Curve 11 shows the fraction of high molecular weight humic substances as a function of exposure time, curve 12 - the color index as a function of exposure time. One can see from Fig. 3 that as the ultrasonic exposure time for the given wave irradiation parameters increases, the fraction of high molecular weight humic substances systematically decreased from 18% to 1.5%, whereas the color index first drops from 0.7 to 0.35 in the interval from 1 to 30 minutes, then increases again to 0.69. Thus, the best dispersal effect for the given frequency and power is achieved in the exposure time range from 10 to 30 minutes.

**Example 4.**

The preparations were produced in the same manner as in Examples 1 or 2, except for the regimes of ultrasonic exposure. The treatment was conducted with the power density of 5 W/cm$^3$ and the frequency of 66 kHz. The exposure time varied from 2 to 30 seconds. The molecular weight and color index of the substance produced were assessed as described in Example 3. The dependence of the properties of humic substances in the preparation on the duration of ultrasonic exposure at the power density of 5 W/cm$^2$ and the frequency of 66 kHz is presented in Fig. 4. Curve 13 illustrates the dependence of the fraction of high molecular weight humic substances on the exposure time, curve 14 - the dependence of color index of the solution on the exposure time for the given wave irradiation parameters.

As the ultrasonic exposure times at the frequency of 66 kHz and power density of 5 W/cm$^3$ increases, the fraction of high molecular weight humic substances systematically decreases, with the optimal values of the color index being observed for exposure times from 5 to 20 seconds.

The result of experiments conducted have demonstrated similar results to be achieved under different regimes of ultrasonic treatment, with the exposure time decreasing with the increase of ultrasound power and frequency.

**Example 5.**

The preparations were produced in a manner similar to the one described in Examples 1 and 2, except for the wave treatment. In this case, dispersal was achieved by microwave treatment at the power of 0.5 kW and frequency of 2.45 GHz, with the temperature maintained in the range of 40-50°C.

The effect of microwave exposure time (for the power of 0.5 kW and the frequency of 2.45 GHz) upon the properties of the preparation is presented in Table 4.

Table 4.

| | Exposure time, minutes | | | | | | | | |
|---|---|---|---|---|---|---|---|---|---|
| | 10 | 20 | 30 | 60 | 90 | 120 | 180 | 240 | 300 |
| $D_{445}/D_{665}$ | 0.74 | 0.65 | 0.50 | 0.46 | 0.44 | 0.45 | 0.50 | 0.68 | 0.76 |
| X, % | 18 | 12 | 8 | 5 | 3 | 3 | 2,5 | 2 | 2 |

It can be seen from the table that microwave treatment of the preparation for 60-120 minutes provides for the best characteristics of the product in terms of color index and fraction of high molecular weight humic substances, and may be used in lieu of ultrasonic treatment.

**Example 6.**

Stability of the preparations produced as described in Examples 3-4 and having the optimal molecular weight and color index parameters was studied in "artificial aging" experiments, which involved storage of the preparations at the temperature of 60°C for 45 days. The stability criteria included pH, the content of molecular aggregates (appearing as foreign inclusions under fluorimetric analysis) and changes in the color index. The principal results are presented below.

The changes in pH of the solutions of preparations produced under different variants of the method claimed when stored under "accelerated aging" conditions are presented in Table 5.

The treatment regimes are abbreviated in the table: US for ultrasonic treatment, MW for microwave treatment.

Table 5.

| pH of a solution of the preparation in the course of storage | | | | | |
|---|---|---|---|---|---|
| Preparation, production method | Initial solution | 10 days | 20 days | 30 days | 45 days |
| US 22 kHz 10 minutes | 8.70 | 8.79 | 8.82 | 8.82 | 8.78 |
| US 22 kHz 60 minutes | 8.80 | 8.78 | 8.83 | 8.76 | 8.76 |
| US 66 kHz 5 seconds | 8.80 | 8.80 | 8.81 | 8.80 | 8.82 |
| US 66 kHz 20 seconds | 8.79 | 8.84 | 8.81 | 8.80 | 8.81 |
| MW 2.45 GHz 60 minutes | 8.71 | 8.77 | 8.75 | 8.70 | 8.80 |
| MW 2.45 GHz 120 minutes | 8.75 | 8.81 | 8.78 | 8.84 | 8.80 |
| Prototype | 8.79 | 8.82 | 8.71 | 8.68 | 8.63 |

The number of specimens revealing foreign inclusions under fluorimetric analysis (in a series of 100 specimens) in the course of storage under "accelerated aging" conditions of the preparation specimens produced under different variants of the method claimed is shown in Table 6.

Table 6

| Number of specimens with foreign inclusions (in a series of 100 specimens) | | | | | |
|---|---|---|---|---|---|
| Preparation, production method | Initial solution | 10 days | 20 days | 30 days | 45 days |
| US 22 kHz 10 minutes | None | None | None | 1 | 1 |
| US 22 kHz 60 minutes | None | None | None | None | None |
| US 66 kHz 5 seconds | None | None | 1 | None | 1 |
| US 66 kHz 20 seconds | None | None | None | 1 | None |
| MW 2.45 GHz 60 minutes | None | None | None | None | 1 |
| MW 2.45 GHz 120 minutes | None | None | None | None | None |
| Prototype | 1 | 1 | 2 | 1 | 2 |

The changes in color index of preparation specimens produced by different variants of the method in the course of storage under "accelerated aging" conditions are presented in Table 7.

Table 7.

| $D_{445}/D_{665}$ | | | | | |
|---|---|---|---|---|---|
| Preparation, production method | Initial solution | 10 days | 20 days | 30 days | 45 days |
| US 22 kHz 10 minutes | 0.50 | 0.52 | 0.51 | 0.49 | 0.52 |
| US 22 kHz 60 minutes | 0.45 | 0.44 | 0.46 | 0.48 | 0.48 |
| US 66 kHz 5 seconds | 0.44 | 0.40 | 0.41 | 0.43 | 0.42 |
| US 66 kHz 20 seconds | 0.43 | 0.46 | 0.45 | 0.43 | 0.46 |
| MW 2.45 GHz 60 minutes | 0.46 | 0.47 | 0.51 | 0.53 | 0.49 |
| MW 2.45 GHz 120 minutes | 0.45 | 0.47 | 0.50 | 0.52 | 0.50 |
| Prototype | 0.55 | 0.61 | 0.67 | 0.68 | 0.71 |

The "accelerated aging" test has demonstrated the preparations produced according to the invention to exhibit better stability characteristics (namely, the color index and the occurrence of foreign inclusion) at the end of the accelerated aging period, compared to the prototype. The acidity figures remained approximately the same for the specimens produced by the method claimed and those produced by the prototype method.

The preparation proposed was also found to be active both as a radioprotector (i.e. was efficient when administered before irradiation) and as a therapeutic drug for accelerating excretion of radionuclides from the organism already damaged by radiation, its activity varying towards one or the other, depending on the content of ammonium molybdate.

**Claims**

1.    Method for producing means protecting an organism against ionizing radiations based on humic substances, in which the aqueous solution containing humic substances and ammonium molybdate is treated with wave irradiation, **characterized in that** the content of ammonium molybdate is selected in the range up to 0.4 parts by weight per 1 part of humic substances, the treatment being conducted until the fraction of humic substances with a molecular weight above 10,000 Daltons is decreased to a level of 5% or below.

2.    Method for producing means protecting an organism against ionizing radiations according to claim 1, **characterized in that** the wave radiation is selected in the ultrasonic frequency range from 18 kHz to 66 kHz, with the irradiation power density in the range of 0.5 to 5 W/cm$^3$.

3.  Method for producing means protecting an organism against ionizing radiations according to claim 1, **characterized in that** the treatment is conducted at the irradiation power density of 5 W/cm$^3$ at the frequency of 22 kHz for 5 to 20 minutes.

4.  Method for producing means protecting an organism against ionizing radiations according to claim 1, **characterized in that** the wave radiation is selected in the microwave frequency range from 0.3 kHz to 30 GHz, with the irradiation power in the range of 0.5 to 50 kW.

5.  Method for producing means protecting an organism against ionizing radiations according to claim 4, **characterized in that** the treatment is conducted at the irradiation power density of 5 W/cm$^3$ at the frequency of 2.45 kHz, with the temperature of the product under treatment being maintained in the range of 60-70°C for 30 to 90 minutes.

6.  Method for producing means protecting an organism against ionizing radiations per any of the claims 1-5, **characterized in that** the humic substances are obtained by oxidation of wood lignin.


**Patentansprüche**

1.  Verfahren zum Herstellen eines Mittels als Schutz eines Organismus gegen ionisierende Strahlungen basierend auf humitischen Substanzen, wobei die wässrige Lösung enthaltend humitische Substanzen und Ammoniummolybdat mit Wellenbestrahlung behandelt wird, **dadurch gekennzeichnet, dass** der Gehalt an Ammoniummolybdat ausgewählt wird im Bereich von bis zu 0,4 Gewichtsteilen pro 1 Teil humitische Substanzen, wobei die Behandlung durchgeführt wird, bis die Fraktion an humitischen Substanzen mit einem Molekulargewicht oberhalb von 10.000 Dalton auf ein Niveau von 5% oder weniger abgesenkt ist.

2.  Verfahren zum Herstellen eines Mittels als Schutz eines Organismus gegen ionisierende Strahlungen nach Anspruch 1, **dadurch gekennzeichnet, dass** die Wellenstrahlung ausgewählt wird im Ultraschallfrequenzbereich von 18 kHz bis 66 kHz, wobei die Strahlungsleistungsdichte im Bereich von 0,5 bis 5 W/cm$^3$ liegt.

3.  Verfahren zum Herstellen eines Mittels als Schutz eines Organismus gegen ionisierende Strahlungen nach Anspruch 1, **dadurch gekennzeichnet, dass** die Behandlung bei der Bestrahlungsleistungsdichte von 5 W/cm$^3$ bei der Frequenz von 22 kHz für 5 bis 20 Minuten durchgeführt wird.

4.  Verfahren zum Herstellen eines Mittels als Schutz eines Organismus gegen ionisierende Strahlungen nach Anspruch 1, **dadurch gekennzeichnet, dass** die Wellenstrahlung ausgewählt wird im Mikrowellenfrequenzbereich von 0,3 kHz bis 30 GHz, wobei die Bestrahlungsleistung im Bereich von 0,5 bis 50 kW liegt.

5.  Verfahren zum Herstellen eines Mittels als Schutz eines Organismus gegen ionisierende Strahlungen nach Anspruch 4, **dadurch gekennzeichnet, dass** die Behandlung bei der Bestrahlungsleistungsdichte von 5 W/cm$^3$ bei der Frequenz von 2,45 kHz durchgeführt wird, wobei die Temperatur des Produkts unter Behandlung im Bereich von 60-70°C für 30 bis 90 Minuten gehalten wird.

6.  Verfahren zum Herstellen eines Mittels als Schutz eines Organismus gegen ionisierende Strahlungen nach einem der Ansprüche 1 bis 5, **dadurch gekennzeichnet, dass** die humitischen Substanzen durch Oxidation von Holzlignin erhalten werden.


**Revendications**

1.  Procédé de production de moyens de protection d'un organisme contre des rayonnements ionisants à base de substances humiques, où la solution aqueuse contenant des substances humiques et du molybdate d'ammonium est traitée avec un rayonnement d'ondes, **caractérisé en ce que** la teneur du molybdate d'ammonium est sélectionnée dans la plage allant jusqu'à 0,4 partie en poids pour 1 partie de substances humiques, le traitement étant effectué jusqu'à ce que la fraction de substances humiques avec un poids moléculaire supérieur à 10.000 Daltons soit réduite à un niveau de 5% ou moins.

2.  Procédé de production de moyens de protection d'un organisme contre des rayonnements ionisants selon la revendication 1, **caractérisé en ce que** le rayonnement d'ondes est sélectionné dans la plage de fréquences ultrasonores allant de 18 kHz à 66 kHz, avec la densité de puissance de rayonnement dans la plage allant de 0,5 à 5 W/cm$^3$.

3. Procédé de production de moyens de protection d'un organisme contre des rayonnements ionisants selon la revendication 1, **caractérisé en ce que** le traitement est effectué à la densité de puissance de rayonnement de 5 W/cm$^3$ à la fréquence de 22 kHz pendant 5 à 20 minutes.

4. Procédé de production de moyens de protection d'un organisme contre des rayonnements ionisants selon la revendication 1, **caractérisé en ce que** le rayonnement d'ondes est sélectionné dans la plage des hyperfréquences allant de 0,3 kHz à 30 GHz, avec la puissance de rayonnement dans la plage allant de 0,5 à 50 kW.

5. Procédé de production de moyens de protection d'un organisme contre des rayonnements ionisants selon la revendication 4, **caractérisé en ce que** le traitement est effectué à la densité de puissance de rayonnement de 5 W/cm$^3$ à la fréquence de 2,45 kHz, avec la température du produit sous traitement étant maintenue dans la plage allant de 60 à 70°C pendant 30 à 90 minutes.

6. Procédé de production de moyens de protection d'un organisme contre des rayonnements ionisants selon l'une des revendications 1 à 5, **caractérisé en ce que** les substances humiques sont obtenues par oxydation de la lignine de bois.

# Copper levels in the blood of mice, µg/g

Sampling time

Fig. 1

Copper levels in the urine of mice, µg/g

Sampling time

Fig. 2

Treatment duration, minutes

Fig. 3

Treatment duration, seconds

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 208898

- RU 2183124

**Non-patent literature cited in the description**

- **E.N. GONCHARENKO ; YU.B. KUDRYASHOV.** Chemical protection against radiation injury. Moscow. *Moscow State University,* 1985, 147